# EUROPEAN PATENT SPECIFICATION

(11) **EP 4 018 981 B1**
(45) Date of publication and mention of the grant of the patent: **16.08.2023**
(21) Application number: 21216820.7
(22) Date of filing: 22.12.2021
(51) Int. Cl.: A61F 9/008

(54) **OPHTHALMOLOGICAL ULTRA-VIOLET LASER SYSTEM FOR EYE TREATMENT**
OPHTHALMOLOGISCHES UV-LASERSYSTEM ZUR AUGENBEHANDLUNG
SYSTÈME OPHTALMOLOGIQUE LASER ULTRAVIOLET POUR LE TRAITEMENT DE L'OEIL

(30) Priority: 24.12.2020 CH 16722020
(43) Date of publication of application: 29.06.2022
(73) Proprietor: Ziemer Ophthalmic Systems AG, 2562 Port (CH)
(72) Inventor: RATHJEN, Christian, 28197 Bremen (DE); BACHER, Christoph, 3008 Bern (CH)
(74) Representative: Rentsch Partner AG

(56) References cited:
- COUTTS D W ET AL: "Cerium-Doped Fluoride Lasers", IEEE JOURNAL OF QUANTUM ELECTRONICS, IEEE SERVICE CENTER, PISCATAWAY, NJ, USA, vol. 40, no. 10, 1 October 2004 (2004-10-01), pages 1430-1440, XP011119169, ISSN: 0018-9197, DOI: 10.1109/JQE.2004.834775
- SAMUEL P ET AL: "Efficient energy transfer between Ce^3^+ and Nd^3^+ in cerium codoped Nd: YAG laser quality transparent ceramics", JOURNAL OF ALLOYS AND COMPOUNDS, ELSEVIER SEQUOIA, LAUSANNE, CH, vol. 507, no. 2, 5 August 2010 (2010-08-05), pages 475-478, XP027320509, ISSN: 0925-8388 [retrieved on 2010-09-23]

## Description

### Field of the Disclosure

The present disclosure relates to an ophthalmological ultra-violet laser system for eye treatment. In particular, the present disclosure relates to an ophthalmological ultra-violet laser system for surgical treatment an eye using a pulsed laser beam.

### Background of the Disclosure

Ultra-violet (UV) lasers for eye treatment are used in many different types of eye treatment, for example refractive surgery in which the cornea of the eye is ablated. By using a pulsed laser with an adjustable repetition rate and energy per pulse, it is possible to provide sufficient energy to ablate targeted tissue without causing excess heating in surrounding tissues. A well-known type of laser used for eye treatment is an excimer laser, which uses a combination of a noble gas and a reactive gas under high pressure and electrical stimulation. For eye treatment, excimer lasers using argon as the noble gas and fluoride as the reaction gas are often used, as the laser wavelength produced is 193 nm which has been found suitable for eye treatment. Among the disadvantages of excimer lasers are that they are often large and bulky and that the use of high pressure gasses presents numerous technical challenges.

Nd:YAG is a crystal that is used as a lasing medium for solid state lasers, typically emitting in the infra-red. Other varieties of YAG are known for their lasing properties, including Er:YAG and Yb:YAG, and many other varieties of YAG are in use in cathode ray tubes, LEDs, or temperature sensing devices.

The article "Cerium-Doped Fluoride Lasers" by COUTTS D W ET AL, IEEE JOURNAL OF QUANTUM ELECTRONICS, vol. 40, no. 10, p. 1430-1440 (1. October 2004) teaches a non-ophthalmological Ce:YAG laser system.

### Summary of the Disclosure

It is an object of the present disclosure to propose an ophthalmological ultra-violet laser system for eye treatment, which does not have at least some of the disadvantages of the prior art. Particularly, it is an object of the present disclosure to propose an ophthalmological ultra-violet laser system for eye treatment which is compact and efficient and produces a more biologically suitable wavelength.

According to the present disclosure, these objects are achieved by the features of the independent claims. Moreover, further advantageous embodiments emerge from the dependent claims and the description.

An ophthalmological ultra-violet (UV) laser system for eye treatment is proposed. The ophthalmological UV laser system comprises laser source including a Ce:YAG (cerium doped yttrium aluminium garnet) gain medium configured to generate a pulsed primary laser beam, as well as a frequency converter arranged downstream from the laser source comprising one or more non-linear optical crystals configured to multiply the frequency of the primary laser beam to generate a pulsed UV laser beam.

In an embodiment, the laser source is a solid-state laser source including the Ce:YAG gain medium configured to generate the pulsed primary laser beam.

In an embodiment, the laser source is an optical fiber laser source including a Ce:YAG gain medium configured to generate the pulsed primary laser beam.

In an embodiment, the ophthalmological UV laser system further comprises a positioning module arranged downstream of the frequency converter, which frequency converter is configured to position the UV laser beam such that the UV laser beam enters an eye of a patient.

In an embodiment, the positioning module comprises a fiber-optic element configured to guide the UV laser beam directly into the eye.

In an embodiment, the positioning module is configured to position the UV laser beam such that the UV laser beam is focused onto tissue of the eye.

In an embodiment, the frequency converter arranged downstream from the laser source comprises a non-linear optical crystal configured to double the frequency of the primary laser beam.

In an embodiment, the frequency converter arranged downstream from the laser source comprises two non-linear optical crystals configured to triple the frequency of the primary laser beam.

In an embodiment, the Ce:YAG crystal is configured such that the primary laser beam has a wavelength of one or more of: 600 nm to 660 nm, 615 nm to 645 nm, 630 nm to 639 nm, or 614 nmto 618 nm.

In an embodiment, the laser source is configured such that the pulsed UV laser beam has a pulse energy of 0.5 mJ to 2.0 mJ.

In an embodiment, the laser source is configured such that the pulse UV laser beam has a power of 0.5 W to 2 W.

In an embodiment, the laser source is configured such that the pulsed UV laser beam, in particular a pulse energy and a wavelength of the pulsed UV laser beam, generated by the frequency converter results in tissue of a patient being ablated by less than 2 µm, preferably 0.5 µm to 1 µm, per pulse of the UV laser beam.

In an embodiment, the laser source comprises an active Q-switch configured to periodically modulate a Q-factor of the laser source such that the primary laser beam has a pulse repetition rate of one or more of: 10 Hz to 1000 Hz or 250 Hz to 1000 Hz.

In an embodiment, the laser source includes a plurality of blue laser diodes arranged around the Ce:YAG crystal as laser pumps.

In an embodiment, the ophthalmological UV laser system further comprises a beam shaper arranged downstream from the frequency converter comprising one or more optical elements and configured to shape the UV laser beam such that the shaped UV laser beam has a fluence of one or more of: 50 mJ/cm² to 400 mJ/cm² or 150 mJ/cm² to 250 mJ/cm2 on the tissue of the patient.

In an embodiment, the ophthalmological UV laser system further comprises a scanner arranged downstream of the frequency converter configured to direct the UV laser beam onto a plurality of positions on a tissue of a patient according to a pre-determined pattern.

In an embodiment, the ophthalmological UV laser system further comprises a wavelength selector arranged downstream of the frequency converter configured to allow a particular wavelength range of the UV laser beam to pass while redirecting a remainder of the UV laser beam onto a beam dump.

In an embodiment, the ophthalmological UV laser system further comprises a pulse diagnostics module arranged downstream of the frequency converter configured to measure a power of the UV laser beam.

### Brief Description of the Drawings

The present disclosure will be explained in more detail, by way of example, with reference to the drawings in which:
- Figure 1:: shows a block diagram that schematically illustrates an ophthalmological ultra-violet (UV) laser system for eye treatment.
- Figures 2-3:: show block diagrams of ophthalmological ultra-violet laser systems for eye treatment including a solid-state laser source.
- Figures 4-5:: show block diagrams of ophthalmological ultra-violet laser systems for eye treatment including a mode-locked laser source.
- Figures 6-8:: show block diagrams of ophthalmological ultra-violet laser systems for eye treatment including an optical fiber laser source.
- Figures 9-10:: show block diagrams of ophthalmological ultra-violet laser systems for eye treatment including an optical fiber ring laser source.
- Figures 11-12:: show block diagrams of ophthalmological ultra-violet laser systems for eye treatment including further modules downstream from the laser source.

### Detailed Description of the Embodiments

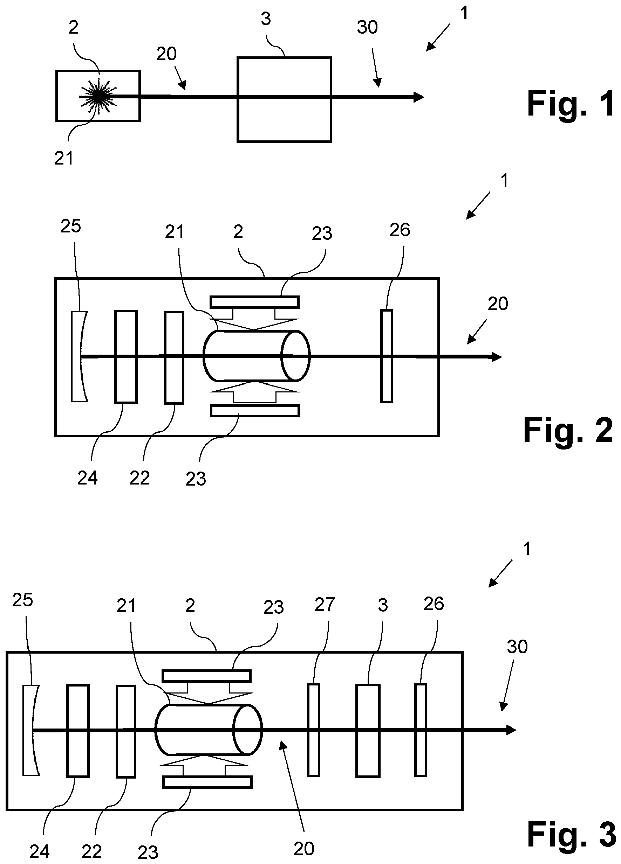

In **Figure 1**, reference numeral 1 relates to an ophthalmological ultra-violet (UV) laser system for eye treatment. A laser source 2 comprises a Ce:YAG (cerium doped yttrium aluminium garnet) gain medium 21 . The laser source 2 generates a primary laser beam 20 in a wavelength range of 600 nm to 750 nm. While Ce:YAG single-crystal phosphors are known in the art of light emitting diodes, particularly for producing high-brightness white LEDs, Ce:YAG as a lasing medium lasing in a wavelength range of 600 nm to 750 is not known. Figure 1 and also the remaining figures are block diagrams which schematically illustrate modules and/or elements of various embodiments of the ophthalmological UV laser system 1 and give an exemplary sequence or arrangement of the modules and/or elements in a beam path. Scale and laws of reflection are not depicted or precisely considered.

Depending on the embodiment, the laser source 2 is configured to generate the primary laser beam 20 such that it has a wavelength of one or more of: 600 nm to 660 nm, 615 nm to 645 nm, 630 nm to 639 nm, or 614 nm to 618 nm. The primary laser beam 20 is a pulsed laser beam. The laser source 2 is configured to generate the primary laser beam with a pulse repetition rate of one or more of: 10 Hz to 1000 Hz, or 250 Hz to 1000 Hz. A frequency converter 3 arranged downstream from the laser source 2 increases the frequency of the primary laser beam 20. The frequency converter 3 comprises one or more non-linear optical crystals 31 which multiply the frequency of the primary laser beam 20 by a factor of two or three, depending on the embodiment, to generate a UV laser beam 30, that is, a laser beam 30 with a wavelength in the ultra-violet wavelength range of approximately 100 nm to 400 nm. The UV laser beam 30 has, when the primary laser beam 20 is tripled in frequency, a wavelength of one or more of: 200 nm to 220 nm, 205 nm to 215 nm, or 210 nm to 213 nm. These wavelength ranges have been shown to be particularly advantageous for ablation of collagen tissue. Further, due UV light absorption in humid air which increases at decreasing wavelengths, the wavelength ranges indicated above more efficiently transfer energy through air than, for example, an excimer laser at 193 nm.

The UV laser beam 30 has, when the primary laser beam 20 is doubled in frequency, a range of one or more of: 300 nm to 330 nm, 307 nm to 322 nm, 315 nm to 319 nm, or 307 nm to 309 nm. The UV laser beam 30 is also pulsed and, depending on the embodiment, has the same or a different repetition rate as the primary laser beam 20 which is, depending on the embodiment, 10 Hz to 1000 Hz or 250 Hz to 1000 Hz. The ophthalmological UV laser system 1, in particular the laser source 2 and the frequency converter 3, are configured to generate a UV laser beam 30 of a particular wavelength or wavelength range, depending on the type of eye treatment for which the ophthalmological UV laser system 1 is designed for. The laser source 2 described above is advantageous because it is compact and efficient. It does not occupy much space in an ophthalmological UV laser system 1 in comparison with state of the art systems, in particular because it does not require the use of high temperature and pressure gasses, as is for example required with known excimer lasers. Further, the laser source 2 does not require as much electrical power to run, and also runs more quietly which is a sought-after characteristic.

The ophthalmological UV laser system 1 is particularly flexible in its configuration and, depending on the embodiment, is configured produce one or more of a wide range of wavelengths of the UV laser beam 30, a wide range of pulse repetition rates, pulse durations, and pulse energies. Good results are achieved when the laser source 2 is configured such that the pulsed UV laser beam 30, in particular a pulse energy and a wavelength of the pulsed UV laser beam 30, generated by the frequency converter 3 results in tissue of a patient being ablated by less than 2 µm, preferably 0.5 µm to 1 µm, per pulse of the UV laser beam 30. This results in a treatment of the eye which is both precise because tissue is ablated only on the order of one µm per pulse, and fast, such that a single eye undergoing refractive surgery can usually be treated in less than one minute. Such an ablation depth per pulse is achieved, in an embodiment where the ophthalmological UV laser system 1 is configured such that the pulsed UV laser beam 30 has a pulse energy of 0.5 mJ to 2.0 mJ. This energy range is advantageous as it is used to ablate eye tissue of a patient quickly while also not resulting in excess heat being deposited in the tissue around the ablated tissue. Such an ablation depth per pulse is also achieved in an embodiment where the ophthalmological UV laser system 1, in particular the laser source 2, is configured such that the UV laser beam 30 has a power of 0.5 W to 2 W.

Below, in relation to Figures 2 to 10, a number of embodiments of the ophthalmological UV laser system 1 are described, in particular the laser source 2 including the Ce:YAG gain medium 21.

**Figures 2** and **3** show block diagrams of the solid-state laser source 2. The solid-state laser source 2 is a laser resonator in which the Ce:YAG gain medium 21 is pumped. The Ce:YAG gain medium 21 is pumped optically or electrically. For example, the Ce:YAG gain medium 21 is pumped using one or more flash lamps or arc lamps. In another example, the Ce:YAG gain medium 21 is pumped using laser diodes arranged either on the side of the Ce:YAG gain medium 21, such that pump light generated by the laser diodes has a transverse direction to an axis of the primary laser beam 20 generated by the Ce:YAG gain medium 21, or on the end of the Ce:YAG gain medium 21, such that the pump light is parallel to the axis of the primary laser beam 20.

In an embodiment, the laser diodes are blue laser diodes 23 arranged on the side of the Ce:YAG gain medium 21 as Ce:YAG absorbs blue light particularly efficiently. Preferably, the blue laser diodes 23 are approximately evenly distributed around the Ce:YAG gain medium 21. A particular homogeneous pumping of the Ce:YAG is achieved with an odd number of blue laser diodes 23, in particular 3, 5, or 7, evenly distributed around the Ce:YAG gain medium 21. The laser source 2 further comprises a pulse generating element 22, configured to generate a primary laser beam 20 which has a pulse repetition rate of one or more of: 10 Hz to 1000 Hz or 250 Hz to 1000 Hz. In an example, the pulse generating element comprises an active Q-switch which is configured to periodically modulate a Q-factor of the solid-state laser source (2), in particular to modulate the Q-factor of the solid-state laser source 2. In another example, the pulse generating element is an acousto-optic modulator (AOM), a saturable absorber, or a Chopper. The laser source additionally has a rear cavity mirror 25 and an out-coupling mirror 26 arranged to form a laser resonator together with the Ce:YAG gain medium 21. In particular, the rear cavity mirror 25 and the out-coupling mirror 26 are arranged in a beam path of the primary laser beam 20, the rear cavity mirror 25 reflecting the entire primary laser beam 20 and the out-coupling mirror 26 reflecting a part of the primary laser beam 20 back into the laser resonator and coupling another part of the primary laser beam 20 out of the laser resonator. A wavelength tuner 24 is arranged in the laser source 20 either between the Ce:YAG gain medium 21 and the rear cavity mirror 25 or between the Ce:YAG gain medium 21 and the out-coupling mirror 26. The wavelength tuner 24 filters primary laser beam 20, allowing only a limited range (band) of wavelengths to pass through. The wavelength tuner 24 is used to configure the laser source 2 to emit one or more of the wavelength ranges described above. In an embodiment, the wavelength tuner 24 includes a bandpass filter, Etalon, and/or a dichroic mirror.

**Figure 3** shows a block diagram of the laser source 2 additionally comprising the frequency converter 3 such that the laser source 2 directly emits the UV laser beam 30. The frequency converter 3 is arranged in the beam path of the primary laser beam 20 between a coated mirror 27 and the out-coupling mirror 26. The coated mirror 27 is a mirror which reflects or transmits a specific wavelength of light with a particular ratio. The particular ratio for a given wavelength is specified by the particular coating of the coated mirror 27 and depends on the embodiment, in particular the configuration of the wavelength tuner 24. The advantage of integrating the frequency converter 3 directly into the laser source 2, in particular directly into the laser resonator, is to allow for a more compact overall ophthalmological UV laser system 1. Further benefits include improved commissioning and servicing of the ophthalmological UV laser system 1, as the laser source 2 and the frequency converter 3 are, for example, no longer required to be independently commissioned and validated, but may be tested and integrated as a single unit of specified performance.

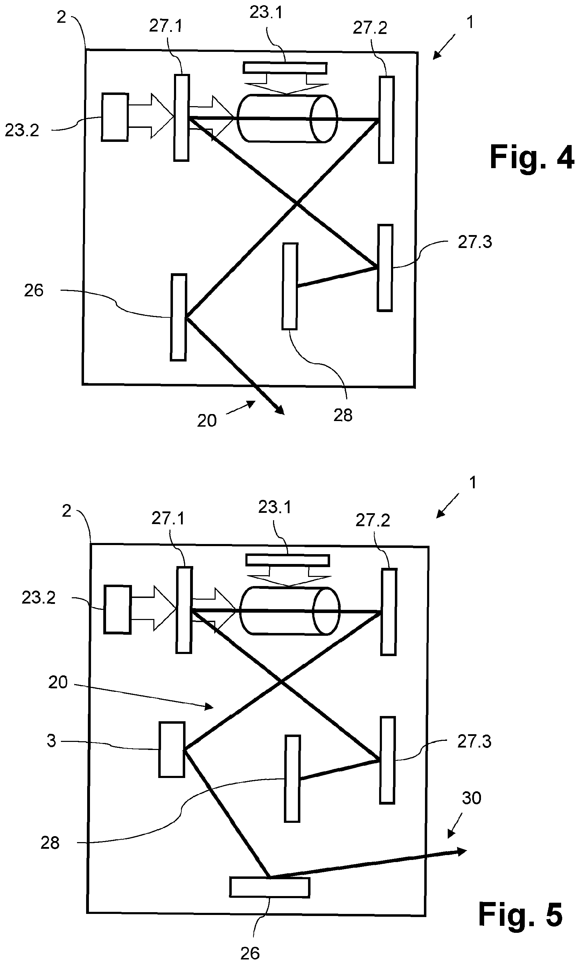

**Figures 4** and **5** show two block diagrams of embodiments of a mode-locked laser source 2 including the Ce:YAG gain medium 21. In these embodiments, the Ce:YAG gain medium 21 is pumped side-on, as shown by the blue laser diodes 23.1 arranged to pump the Ce:YAG gain medium 21 perpendicular to the axis of the primary laser beam 20 within the Ce:YAG gain medium 21, and/or end-on, as shown by the blue laser diodes 23.2 arranged to pump the Ce:YAG gain medium 21 on an axis parallel to the axis of the primary laser beam 20. Two coated mirrors 27.1, 27.2 are arranged on either side of the Ce:YAG gain medium 21. A first coated mirror 27.1 is configured to redirect the primary laser beam 20 exiting the Ce:YAG gain medium 21 to a third coated mirror 27.3, which in turn redirects the primary laser beam 20 to a saturable mirror 28. In an embodiment, the third coated mirror 27.3 is omitted and replaced by the saturable mirror 28. The saturable mirror is configured for mode-locking the laser source 2 and producing a pulsed primary laser beam 20 and is, for example, a semiconductor saturable-absorber mirror (SESAM). the second coated mirror 27.2 is configured to redirect the primary laser beam 20 to the out-coupling mirror 26 which is configured to allow a portion of the primary laser beam 20 to exit the laser source 2.

**Figure 5** shows a block diagram of a laser source 2 in which the frequency converter 3 is arranged between the second coated mirror 27.2 and the out-coupling mirror 26 and generates, from the primary laser beam 20, the UV laser beam 30 which exits the laser source 2 through the out-coupling mirror 26. The advantage of integrating the frequency converter 3 into the laser source 2 is similar as described above, namely achieving better integration and thereby reducing the overall system complexity.

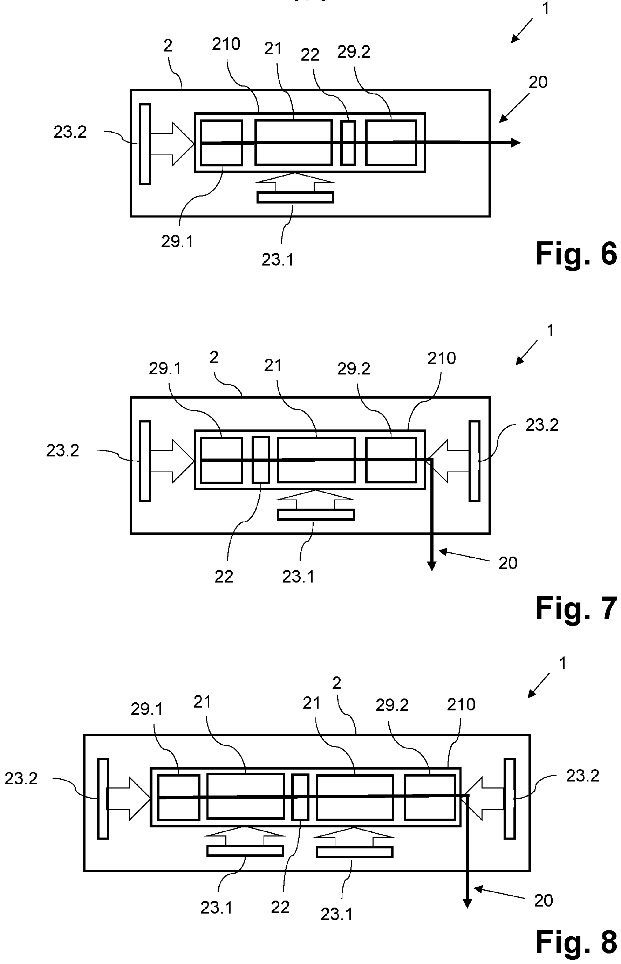

**Figures 6** to **8** show block diagrams of various embodiments of laser sources 2 as an optical fiber 210, in particular in which the Ce:YAG gain medium 21 is part of the optical fiber 210. The Ce:YAG gain medium 21 is pumped side-on (perpendicular to a central axis of the optical fiber 210), for example by blue laser diodes 23.1, and/or end-on (parallel to the central axis of the optical fiber 210), for example by blue laser diodes 23.2. In Fig. 6, an embodiment is shown in which the optical fiber 210 receives pump light both from side-on laser diodes 23.1 and from end-on laser diodes 23.2. The optical fiber 210 includes the Ce:YAG gain medium 21 as well as two fiber Bragg gratings (FBG) 29.1 and 29.2 arranged in the optical fiber 210 on both sides of the Ce:YAG gain medium 21. The FBGs 29 are perturbations in the optical fiber 210 configured to be reflective to the primary laser beam 20. The first FBG 29.1 arranged on the same side of the Ce:YAG gain medium 21 as the end-on blue laser diode 23.2 is configured to be highly reflective. The second FBG 29.2 arranged on the other side of the of the Ce:YAG gain medium 21 is configured to be partially reflective and therefore to allow part of the primary laser beam 20 to exit the optical fiber 210 and the laser source 2. The optical fiber 210 also includes the pulse generating element 22. In an embodiment, the pulse generating element 22 is arranged in between the Ce:YAG gain medium 21 and the FBG 29.2.

**Figure 7** shows a block diagram of an optical fiber laser source 2 which includes a first blue laser diode 23.2 configured to pump the optical fiber 210, in particular the Ce:YAG gain medium 21, and which further includes a second end-on blue laser diode 23.2 on the other end of the optical fiber 210 to the first blue laser diode 23.2, such that the Ce:YAG gain medium 21 is pumped from both ends. Two FBGs 29.1 and 29.2 are arranged in the optical fiber 210 on either side of the Ce:YAG gain medium 21, wherein the first FBG 29.1 is configured to be highly reflective and the second FBG 29.2 is configured to be partially reflective. The primary laser beam 20 is coupled out of the optical fiber 210 downstream of the second FBG 29.2 using an out-coupling module (not shown).

**Figure 8** shows a block diagram illustrating schematically another embodiment of an optical fiber laser source 2 in which the optical fiber 210 is pumped on both ends using the blue laser diodes 29.1, 29.2 arranged as described above. Further, two regions of the optical fiber 210 include a Ce:YAG gain medium 21, the pulse generating element 22 being arranged in the optical fiber 210 between the two regions which include the Ce:YAG gain medium 21. Each region which includes the Ce:YAG gain medium 21 is pumped by blue laser diodes 23.1. This configuration of the optical fiber 210 results in a compact and powerful laser source 2.

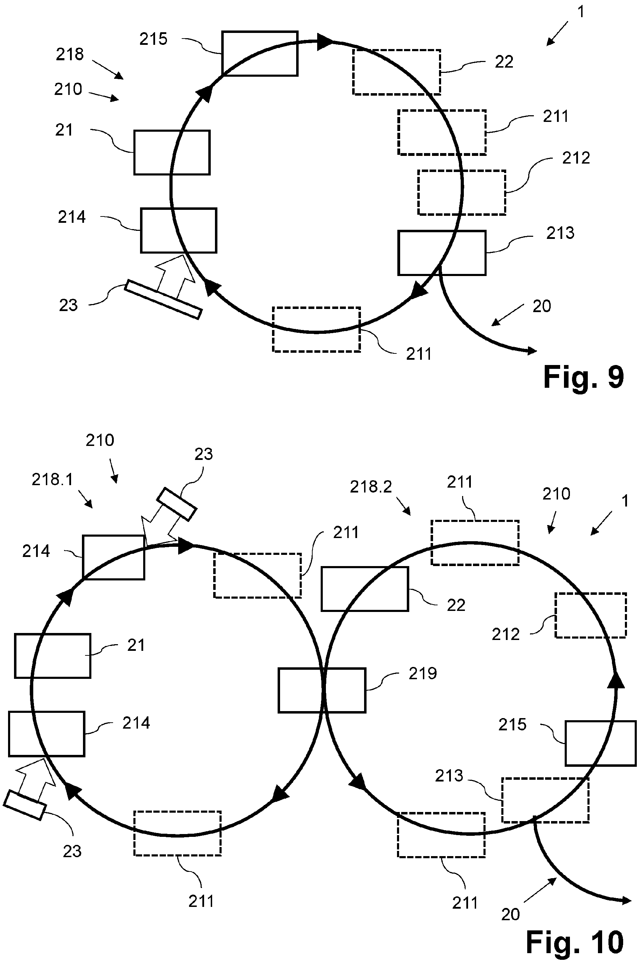

**Figures 9** and **10** show block diagrams show embodiments of the laser source 2 as an optical fiber ring resonator 218, in which unidirectional propagation of the primary laser beam 20 through one or more ring shaped optical fibers 210 is achieved using an optical isolator 21 5 which allows propagation of the primary laser beam 20 only in the direction indicated in the Figs., i.e. clockwise in the optical fiber ring resonator 218 shown in Fig. 9, and clockwise in a first optical fiber ring resonator 218.1 shown in Fig. 10, and anticlockwise in a second optical fiber ring resonator 218.2 in Fig. 10. The first optical fiber ring resonator 218.1 and the second optical fiber ring resonator 218.2 of Fig. 10 are coupled using a fiber coupler 219 which partially couples the primary laser beam 20 from the first optical fiber ring resonator 218.1 into the second optical fiber ring resonator 218.2, and vice-versa. Modules and/or elements of the optical fiber ring resonators 218, 218.1, 218.2 described herein are coupled to the optical fiber 210 and/or integrated into the optical fiber 210 itself. The optical fiber ring resonators 218, 218.1, 218.2 shown in Figs 9 and 10 comprises one or more pump and signal combiners 214 which combine the pump light from one or more blue laser diodes 23 with the primary laser beam 20 circulating in the optical fiber ring resonators. The pump and signal combiners 214include, for example, one or more wavelength division multiplexers. The pump and signal combiners 214 are configured to pump the Ce:YAG gain medium 21 arranged in the optical fiber 210 either from one end or from both ends. The optical isolator 215 is arranged downstream from the Ce:YAG gain medium 21, that is, it is directly adjacent to the Ce:YAG gain medium 21 in the direction of the primary laser beam 20 indicated. An out-coupling element 213 is arranged downstream from the Ce:YAG gain medium 21, preferably downstream of the optical isolator 215, and is configured to couple a portion of the primary laser beam 20 out of the optical fiber ring resonator 218, 218.1, 218.2.

The optical fiber ring resonator 218, 218.1, 218.2 comprises, depending on the embodiment, further modules and/or elements. In an embodiment, the optical fiber ring resonator 218, 218.1, 218.2 comprises one or more polarization controllers 211 arranged as indicated in the Figs, in particular arranged between the optical isolator 215 and the out-coupling element 213, and/or between the out-coupling element 213 and the pump and signal combiner 214. The polarization controllers 211 are configured to control a polarization orientation and/or a shape of the primary laser beam 20. In Fig. 10, additionally to polarization controllers 211 arranged in the first optical fiber ring resonator 218.1, further polarization controllers 211 are arranged, in an embodiment, in the second optical fiber ring resonator 218.2, in particular upstream of the pulse generating element 22 and/or downstream of the fiber coupler 219. Additionally, depending on the embodiment, a wavelength selector 212 is also arranged in the optical fiber ring resonator 218, 218.1, 218.2, preferably downstream from the optical isolator 215.

The skilled person knows that one or more of the above modules, components, or regions of the laser source 2 described above can be reordered, re-arranged, or omitted without deviating from the scope of the invention.

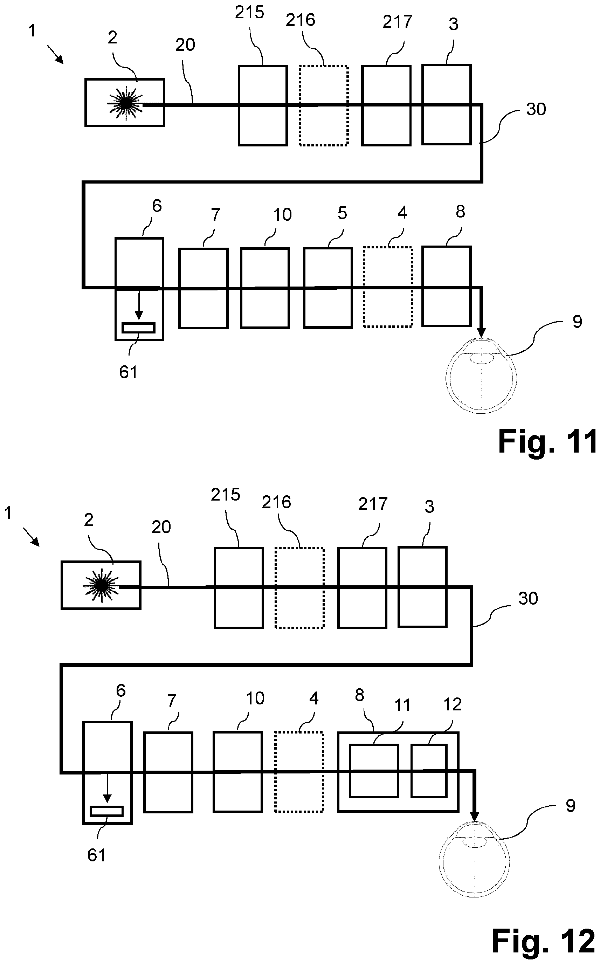

**Figures 11** and **12** show block diagrams of the ophthalmological UV laser system 1 in which the primary laser beam 20 and, in some embodiments, also the UV laser beam 30 are generated in the laser source 2. Depending on the embodiment, some or more of the modules of the ophthalmological UV laser system 1 described below are integrated directly into the laser source 2 (see the descriptions of Figs. 1 to 10 above) or arranged separately from the laser source 2.

Figure 11 is related to an embodiment of the ophthalmological UV laser system 1 in which a beam deflection module 5 is used to deflect the UV laser beam 20 onto and/or into an eye 9 of a patient according to a pre-determined pattern. Figure 12 is related to an embodiment of the ophthalmological UV laser system 1 in which a hand piece 12 is used to manually direct the UV laser beam 30 onto a treatment area in or on the eye 9 of the patient. The embodiments of the ophthalmological UV laser system 1 described herein with relation to Figs. 11 and 12 have many modules and/or elements in common and these may be arranged in a similar order along a beam path of the primary laser beam 20 and/or the UV laser beam 30.

Depending on the embodiment, the primary laser beam 20 travels through free space (i.e. air or a vacuum), and/orthrough an optical medium, such as an optical fiber, between the modules and/or elements.

The ophthalmological UV laser system 1 comprises a laser system controller (not shown) which is configured to control the ophthalmological UV laser system 1. The laser system controller is configured to ensure the safe and reliable operation of the ophthalmological UV laser system 1. The laser system controller is connected to the laser source 2, as well as to at least some further modules and/or elements of the ophthalmological UV laser system 1. The laser system controller is configured to receive input commands from an operator of the ophthalmological UV laser system 1, to receive status data regarding a current status of the ophthalmological UV laser system 1, including parameters of the primary laser beam 20 and/or the UV laser beam 30, and to send commands to the laser source 2 and one or more other modules and/or elements of the ophthalmological UV laser system 1. In an embodiment, the laser system controller comprises a processor configured to execute program instructions.

In an embodiment, the ophthalmological UV laser system 1 comprises an optical isolator 215 arranged downstream (i.e. in a direction of propagation of the primary laser beam 20) of the laser source 2. Depending on a configuration of the ophthalmological UV laser system 1, the optical isolator 215 is not required. The optical isolator 215 is configured such that the primary laser beam 20, or the UV laser beam 30 generated further downstream, cannot travel upstream through the optical isolator 215. Downstream from the optical isolator 215, an optional laser amplifier 216 is arranged. The laser amplifier 216 comprises a further Ce:YAG gain medium 21 which is pumped from further blue laser diodes 23 (not shown). Further downstream, or also optionally upstream of the laser amplifier 216, a pulse picker 217 is arranged. The pulse picker 217 is configured to pick a single or multiple pulses from the primary laser beam 20, in particular from a pulse train of the primary laser beam 20 generated by the laser source 2. The pulse picker 217 reduces the pulse repetition rate of the primary laser beam 20. The pulse picker 217 includes, in an example, a Pockels cell configured for time-dependent modulation of an intensity of the primary laser beam 20 such that a single or multiple pulses are allowed to pass through the pulse picker 217 while other pulses are prevented from passing through the pulse picker 217. The ophthalmological device 1 is designed such that the pulse generating element 22 of the laser source 2 and/or the pulse picker 217 generate a pulsed primary laser beam 20 with a pulse repetition rate of one or more of: 10 Hz to 1000 Hz or 250 Hz to 1000 Hz.

A frequency converter 3 is arranged downstream from the laser source 2, in particular downstream from the pulse picker 217, and configured to double or triple the frequency of the primary laser beam 20 to generate the UV laser beam 30. The frequency converter 3 comprises one or more non-linear crystals 31. In an embodiment, for generating the UV laser beam 30 by doubling the frequency of the primary laser beam 20, a single non-linear crystal 31 which generates a second-harmonic of incoming light may be used. In another embodiment, for generating the UV laser beam 30 by tripling the frequency of the primary laser beam 20, a first non-linear crystal 31 is used which generates a second-harmonic may be used followed by a second non-linear crystal 31 which generates a sum of the primary laser beam 20 and the second-harmonic to generate the UV laser beam 30 with a tripled frequency of the primary laser beam 20. Examples of such non-linear crystals include lithium triborate (LBO) and beta barium borate (BBO) and are well-known in the art.

A wavelength selector 6 is arranged downstream from the frequency converter 3. The wavelength selector 6 is configured to select a narrow range of wavelengths of the UV laser beam 30 such that only a portion of the UV laser beam 30 corresponding to the narrow range passes onward through the wavelength selector 6, a remaining portion of the UV laser beam 30 being redirected in the wavelength selector 6 onto a beam dump 61 configured to absorb and dissipate the remaining portion of the UV laser beam 30. The wavelength selector 6 includes, depending on the embodiment, a dichroic mirror, and/or a FBG in combination with the optical fiber ring resonator 218 described above.

A pulse diagnostics module 7 is arranged downstream from the frequency converter 3. In an embodiment, the pulse diagnostics module 7 is arranged directly downstream from the wavelength selector 6. The pulse diagnostics module 7 is configured to measure a power of the UV laser beam 30. The pulse diagnostics module 7 ensures the safe operation of the ophthalmological UV laser system 1. The ophthalmological UV laser system 1, in particular the laser system controller, is configured to shut off the UV laser beam 30 if a property of UV laser beam 30 is not within specification, for example by closing a shutter 10 and/or powering down the laser source 2. The shutter 10 is arranged downstream of the frequency converter 3, for example downstream of the diagnostics module 7, as shown in Fig. 11. The shutter 10, in an embodiment, is arranged upstream of the diagnostics module 7, as shown in Fig. 12.

The beam deflection module 5 is arranged downstream of the frequency converter 3, in particular downstream of the shutter 10 and is configured to direct the UV laser beam 30 onto a plurality of positions in or on the eye according to the pre-determined pattern. The pattern is, in an embodiment, two-dimensional. In an embodiment, the pattern is three-dimensional. The beam deflection module 5 includes, depending on the embodiment, tilting optics and/or scanning optics. For example, a rotating mirror scanner, a polygon scanner, a galvanometer scanner, an acousto-optic scanner, and/or an electro-optic scanner is used depending on the embodiment.

Depending on the embodiment, a beam shaper 4 is arranged downstream from the frequency converter 3. The beam shaper 4 comprises one or more optical elements and is configured to shape the UV laser beam 30 such that the shaped UV laser beam 40 has a fluence of one or more of: 50 mJ/cm² to 400 mJ/cm² or 150 mJ/cm² to 250 mJ/cm2 on the tissue of the patient. The optical elements comprise, for example, a focusing optic, such as an optical lens, and/or mirrors (flat and/or curved). Depending on the embodiment, the beam shaper 4 is configured to produce a Gaussian shaped UV laser beam 40, Supergaussian shaped UV laser beam 40, and/or a tophat shaped UV laser beam 40.

A positioning module 8 is arranged downstream from the frequency converter 3. In an embodiment, the positioning module 8 is configured to position the UV laser beam 30 such that the UV laser beam 30 enters the eye 9 of the patient. Depending on the embodiment, the positioning module comprises a patient interface which fixes a relative position of the eye 9 to the ophthalmological UV laser system or alternatively an eye tracking module that sends position information of the eye to the scan module in order to correct for eye movement 1 such that the UV laser beam 30 is always positioned correctly during treatment.

In an embodiment shown for example in Fig. 12, the positioning module 8 further comprises an optical waveguide coupler 11 which couples the UV laser beam 30 into a fiber optic element of the positioning module 8. A hand piece 12 is used by the operator to direct the UV laser beam 30 onto or into the eye 9 of the patient manually. In this embodiment, the scanner 5 is not always required. In this embodiment, a lower pulse repetition rate range of 10 Hz to 1000 Hz is used. This embodiment of the ophthalmological UV laser system 1 is used, for example, in eye treatments for which the UV laser beam 30 is required to be absorbed by tissue inside the eye 9. The fiber optic element is passed through a perforation in the eye 9 and an end of the fiber optic element inserted into the eye 9 is juxtaposed to the tissue to be treated. The pulsed UV laser beam 30 then ablates the target tissue, for example a, trabecular meshwork, vitreous membranes, or tear duct occlusion.

## Claims

1. Ophthalmological ultra-violet (UV) laser system (1) for eye treatment comprising:
a. A laser source (2) including a Ce:YAG gain medium (21) configured to generate a pulsed primary laser beam (20); and
b. A frequency converter (3) arranged downstream from the laser source (1) comprising one or more non-linear optical crystals (31) configured to multiply the frequency of the primary laser beam (20) to generate a pulsed UV laser beam (30).

2. The ophthalmological UV laser system (1) of claim 1, wherein the laser source is a solid-state laser source (2) including the Ce:YAG gain medium (21) configured to generate the pulsed primary laser beam (20).

3. The ophthalmological UV laser system (1) of claim 1, wherein the laser source is an optical fiber laser source (2) including a Ce:YAG gain medium (21) configured to generate the pulsed primary laser beam (20).

4. The ophthalmological UV laser system (1) of one of claims 1 to 3, further comprising a positioning module (8) arranged downstream of the frequency converter (3) and configured to position the UV laser beam (30) such that the UV laser beam (30) enters an eye (9) of a patient.

5. The ophthalmological UV laser system (1) of one of claims 1 to 4, wherein the positioning module (8) comprises a fiber-optic element configured to guide the UV laser beam (30) directly into the eye (9).

6. The ophthalmological UV laser system (1) of one of claims 1 to 4, wherein the positioning module (8) is configured to position the UV laser beam (30) such that the UV laser beam (30) is focused onto tissue of the eye (9).

7. The ophthalmological UV laser system (1) of one of claims 1 to 6, wherein the frequency converter (3) arranged downstream from the laser source (1) comprises a non-linear optical crystal (31) configured to double the frequency of the primary laser beam (20).

8. The ophthalmological UV laser system (1) of one of claims 1 to 7, wherein the frequency converter (3) arranged downstream from the laser source (1) comprises two non-linear optical crystals (31) configured to triple the frequency of the primary laser beam (20).

9. The ophthalmological UV laser system (1) of one of claims 1 to 8, wherein the Ce:YAG crystal is configured such that the primary laser beam (20) has a wavelength of one or more of: 600 nm to 660 nm, 615 nm to 645 nm, 630 nm to 639 nm, or 614 nm to 618 nm.

10. The ophthalmological UV laser system (1) of one of claims 1 to 9, wherein the laser source (2) is configured such that the pulsed UV laser beam (30) has a pulse energy of 0.5 mJ to 2.0 mJ.

11. The ophthalmological UV laser system (1) of one of claims 1 to 10, wherein the laser source (2) is configured such that the pulsed UV laser beam (30) has a power of 0.5 W to 2 W.

12. The ophthalmological UV laser system (1) of one of claims 1 to 11, wherein the laser source (2) is configured such that the pulsed UV laser beam (30), in particular a pulse energy and a wavelength of the pulsed UV laser beam (30), generated by the frequency converter (3) results in tissue of a patient being ablated by less than 2 µm, preferably 0.5 µm to 1 µm, per pulse of the UV laser beam (30).

13. The ophthalmological UV laser system (1) of one of claims 1 to 12, wherein the laser source (2) comprises an active Q-switch configured to periodically modulate a Q-factor of the laser source (2) such that the primary laser beam (20) has a pulse repetition rate of one or more of: 10 Hz to 1000 Hz or 250 Hz to 1000 Hz.

14. The ophthalmological UV laser system (1) of one of claims 1 to 13, wherein the laser source (2) includes a plurality of blue laser diodes (23) arranged around the Ce:YAG crystal (21) as laser pumps.

15. The ophthalmological UV laser system (1) of one of claims 1 to 14, further comprising a beam shaper (4) arranged downstream from the frequency converter (3) comprising one or more optical elements (41) and configured to shape the UV laser beam (30) such that the shaped UV laser beam (40) has a fluence of one or more of: 50 mJ/cm² to 400 mJ/cm² or 150 mJ/cm² to 250 mJ/cm2 on the tissue of the patient.

16. The ophthalmological UV laser system (1) of one of claims 1 to 15, further comprising a scanner (5) arranged downstream of the frequency converter (3) configured to direct the UV laser beam (30) onto a plurality of positions on a tissue of a patient according to a pre-determined pattern.

17. The ophthalmological UV laser system (1) of one of claims 1 to 16, further comprising a wavelength selector (6) arranged downstream of the frequency converter (3) configured to allow a particular wavelength range of the UV laser beam (30) to pass while redirecting a remainder of the UV laser beam (30) onto a beam dump (61).

18. The ophthalmological UV laser system (1) of one of claims 1 to 17, further comprising a pulse diagnostics module (7) arranged downstream of the frequency converter (3) configured to measure a power of the UV laser beam (30).

## Patentansprüche

1. Ophthalmologisches Ultraviolett(UV)-Lasersystem (1) zur Augenbehandlung, umfassend:
a. eine Laserquelle (2), beinhaltend ein Ce:YAG-Verstärkungsmedium (21), das dafür ausgelegt ist, einen gepulsten primären Laserstrahl (20) zu erzeugen; und
b. einen Frequenzwandler (3), der der Laserquelle (1) nachgeschaltet angeordnet ist und einen oder mehrere nichtlineare optische Kristalle (31) umfasst, die dafür ausgelegt sind, die Frequenz des primären Laserstrahls (20) zum Erzeugen eines gepulsten UV-Laserstrahls (30) zu multiplizieren.

2. Ophthalmologisches UV-Lasersystem (1) nach Anspruch 1, wobei die Laserquelle eine Festkörperlaserquelle (2) ist, die das Ce:YAG-Verstärkungsmedium (21) beinhaltet, das dafür ausgelegt ist, den gepulsten primären Laserstrahl (20) zu erzeugen.

3. Ophthalmologisches UV-Lasersystem (1) nach Anspruch 1, wobei die Laserquelle eine faseroptische Laserquelle (2) ist, die ein Ce:YAG-Verstärkungsmedium (21) beinhaltet, das dafür ausgelegt ist, den gepulsten primären Laserstrahl (20) zu erzeugen.

4. Ophthalmologisches UV-Lasersystem (1) nach einem der Ansprüche 1 bis 3, ferner umfassend ein Positionierungsmodul (8), das dem Frequenzwandler (3) nachgeschaltet ist und dafür ausgelegt ist, den UV-Laserstrahl (30) so zu positionieren, dass der UV-Laserstrahl (30) in ein Auge (9) eines Patienten eintritt.

5. Ophthalmologisches UV-Lasersystem (1) nach einem der Ansprüche 1 bis 4, wobei das Positionierungsmodul (8) ein faseroptisches Element umfasst, das dafür ausgelegt ist, den UV-Laserstrahl (30) direkt in das Auge (9) zu leiten.

6. Ophthalmologisches UV-Lasersystem (1) nach einem der Ansprüche 1 bis 4, wobei das Positionierungsmodul (8) dafür ausgelegt ist, den UV-Laserstrahl (30) so zu positionieren, dass der UV-Laserstrahl (30) auf Gewebe des Auges (9) fokussiert wird.

7. Ophthalmologisches UV-Lasersystem (1) nach einem der Ansprüche 1 bis 6, wobei der der Laserquelle (1) nachgeschaltete Frequenzwandler (3) einen nichtlinearen optischen Kristall (31) umfasst, der dafür ausgelegt ist, die Frequenz des primären Laserstrahls (20) zu verdoppeln.

8. Ophthalmologisches UV-Lasersystem (1) nach einem der Ansprüche 1 bis 7, wobei der der Laserquelle (1) nachgeschaltete Frequenzwandler (3) zwei nichtlineare optische Kristalle (31) umfasst, die dafür ausgelegt sind, die Frequenz des primären Laserstrahls (20) zu verdreifachen.

9. Ophthalmologisches UV-Lasersystem (1) nach einem der Ansprüche 1 bis 8, wobei der Ce:YAG-Kristall so ausgelegt ist, dass der primäre Laserstrahl (20) eine Wellenlänge von einem oder mehreren der folgenden Werte aufweist: 600 nm bis 660 nm, 615 nm bis 645 nm, 630 nm bis 639 nm, oder 614 nm bis 618 nm.

10. Ophthalmologisches UV-Lasersystem (1) nach einem der Ansprüche 1 bis 9, wobei die Laserquelle (2) so ausgelegt ist, dass der gepulste UV-Laserstrahl (30) eine Pulsenergie von 0,5 mJ bis 2,0 mJ aufweist.

11. Ophthalmologisches UV-Lasersystem (1) nach einem der Ansprüche 1 bis 10, wobei die Laserquelle (2) so ausgelegt ist, dass der gepulste UV-Laserstrahl (30) eine Leistung von 0,5 W bis 2 W aufweist.

12. Ophthalmologisches UV-Lasersystem (1) nach einem der Ansprüche 1 bis 11, wobei die Laserquelle (2) so ausgelegt ist, dass der vom Frequenzwandler (3) erzeugte gepulste UV-Laserstrahl (30), insbesondere eine Pulsenergie und eine Wellenlänge des gepulsten UV-Laserstrahls (30), dazu führt, dass Gewebe eines Patienten um weniger als 2 µm, vorzugsweise 0,5 µm bis 1 µm, pro Puls des UV-Laserstrahls (30) abgetragen wird.

13. Ophthalmologisches UV-Lasersystem (1) nach einem der Ansprüche 1 bis 12, wobei die Laserquelle (2) einen aktiven Q-Schalter umfasst, der dafür ausgelegt ist, einen Q-Faktor der Laserquelle (2) periodisch zu modulieren, derart, dass der primäre Laserstrahl (20) eine Pulswiederholungsrate von einem oder mehreren der folgenden Werte aufweist: 10 Hz bis 1000 Hz oder 250 Hz bis 1000 Hz.

14. Ophthalmologisches UV-Lasersystem (1) nach einem der Ansprüche 1 bis 13, wobei die Laserquelle (2) eine Vielzahl von blauen Laserdioden (23) beinhaltet, die um den Ce:YAG-Kristall (21) als Laserpumpen angeordnet sind.

15. Ophthalmologisches UV-Lasersystem (1) nach einem der Ansprüche 1 bis 14, ferner umfassend einen Strahlformer (4), der dem Frequenzwandler (3) nachgeschaltet angeordnet ist und ein oder mehrere optische Elemente (41) umfasst und dafür ausgelegt ist, den UV-Laserstrahl (30) so zu formen, dass der geformte UV-Laserstrahl (40) eine Fluenz von einem oder mehreren der folgenden Werte aufweist: 50 mJ/cm² bis 400 mJ/cm² oder 150 mJ/cm² bis 250 mJ/cm² auf das Gewebe des Patienten.

16. Ophthalmologisches UV-Lasersystem (1) nach einem der Ansprüche 1 bis 15, ferner umfassend eine Abtastvorrichtung (5), die dem Frequenzwandler (3) nachgeschaltet angeordnet und dafür ausgelegt ist, den UV-Laserstrahl (30) auf eine Vielzahl von Positionen auf einem Gewebe eines Patienten gemäß einem vorbestimmten Muster zu richten.

17. Ophthalmologisches UV-Lasersystem (1) nach einem der Ansprüche 1 bis 16, ferner umfassend einen Wellenlängenselektor (6), der dem Frequenzwandler (3) nachgeschaltet angeordnet und dafür ausgelegt ist, einen spezifischen Wellenlängenbereich des UV-Laserstrahls (30) passieren zu lassen, während er einen Rest des UV-Laserstrahls (30) auf einen Strahlstopper (61) umleitet.

18. Ophthalmologisches UV-Lasersystem (1) nach einem der Ansprüche 1 bis 17, ferner umfassend ein dem Frequenzwandler (3) nachgeschaltetes Pulsdiagnosemodul (7), das dafür ausgelegt ist, eine Leistung des UV-Laserstrahls (30) zu messen.

## Revendications

1. Système laser ophtalmologique à ultraviolet (UV) (1) pour le traitement des yeux comprenant :
a. Une source laser (2) comprenant un milieu de gain Ce:YAG (21) configuré pour générer un faisceau laser primaire pulsé (20) ; et
b. Un convertisseur de fréquence (3) placé en aval de la source laser (1) comprenant un ou plusieurs cristaux optiques non linéaires (31) configurés pour multiplier la fréquence du faisceau laser primaire (20) afin de générer un faisceau laser UV pulsé (30).

2. Système laser UV ophtalmologique (1) selon la revendication 1, dans lequel la source laser est une source laser à état solide (2) comprenant le milieu de gain Ce:YAG (21) configuré pour générer le faisceau laser primaire pulsé (20).

3. Système laser UV ophtalmologique (1) selon la revendication 1, dans lequel la source laser est une source laser à fibre optique (2) comprenant un milieu de gain Ce:YAG (21) configuré pour générer le faisceau laser primaire pulsé (20).

4. Système laser UV ophtalmologique (1) selon l'une des revendications 1 à 3, comprenant en outre un module de positionnement (8) disposé en aval du convertisseur de fréquence (3) et configuré pour positionner le faisceau laser UV (30) de manière à ce que le faisceau laser UV (30) pénètre dans l'œil (9) d'un patient.

5. Système laser UV ophtalmologique (1) selon l'une des revendications 1 à 4, dans lequel le module de positionnement (8) comprend un élément à fibre optique configuré pour guider le faisceau laser UV (30) directement dans l'œil (9).

6. Système laser UV ophtalmologique (1) selon l'une des revendications 1 à 4, dans lequel le module de positionnement (8) est configuré pour positionner le faisceau laser UV (30) de manière à ce que le faisceau laser UV (30) soit focalisé sur le tissu de l'œil (9).

7. Système laser UV ophtalmologique (1) selon l'une des revendications 1 à 6, dans lequel le convertisseur de fréquence (3) disposé en aval de la source laser (1) comprend un cristal optique non linéaire (31) configuré pour doubler la fréquence du faisceau laser primaire (20) .

8. Système laser UV ophtalmologique (1) selon l'une des revendications 1 à 7, dans lequel le convertisseur de fréquence (3) disposé en aval de la source laser (1) comprend deux cristaux optiques non linéaires (31) configurés pour tripler la fréquence du faisceau laser primaire (20).

9. Système laser UV ophtalmologique (1) selon l'une des revendications 1 à 8, dans lequel le cristal Ce:YAG est configuré de telle sorte que le faisceau laser primaire (20) a une longueur d'onde situé dans une ou plusieurs des plages suivantes : de 600 nm à 660 nm, de 615 nm à 645 nm, de 630 nm à 639 nm, ou de 614 nm à 618 nm.

10. Système laser UV ophtalmologique (1) selon l'une des revendications 1 à 9, dans lequel la source laser (2) est configurée de telle sorte que le faisceau laser UV pulsé (30) a une énergie d'impulsion de 0,5 mJ à 2,0 mJ.

11. Système laser UV ophtalmologique (1) selon l'une des revendications 1 à 10, dans lequel la source laser (2) est configurée de sorte que le faisceau laser UV pulsé (30) a une puissance de 0,5 W à 2 W.

12. Système laser UV ophtalmologique (1) selon l'une des revendications 1 à 11, dans lequel la source laser (2) est configurée de sorte que le faisceau laser UV pulsé (30), en particulier une énergie d'impulsion et une longueur d'onde du faisceau laser UV pulsé (30), généré par le convertisseur de fréquence (3) entraîne l'ablation des tissus d'un patient de moins de 2 µm, de préférence de 0,5 µm à 1 µm, par impulsion du faisceau laser UV (30) .

13. Système laser UV ophtalmologique (1) selon l'une des revendications 1 à 12, dans lequel la source laser (2) comprend un commutateur Q actif configuré pour moduler périodiquement un facteur Q de la source laser (2) de sorte que le faisceau laser primaire (20) a une fréquence de répétition des impulsions située dans une ou plusieurs des plages suivantes : de 10 Hz à 1000 Hz ou de 250 Hz à 1000 Hz.

14. Système laser UV ophtalmologique (1) selon l'une des revendications 1 à 13, dans lequel la source laser (2) comprend une pluralité de diodes laser bleues (23) disposées autour du cristal Ce:YAG (21) comme pompes laser.

15. Système laser UV ophtalmologique (1) selon l'une des revendications 1 à 14, comprenant en outre un dispositif de mise en forme du faisceau (4) disposé en aval du convertisseur de fréquence (3) comprenant un ou plusieurs éléments optiques (41) et configuré pour mettre en forme le faisceau laser UV (30) de sorte que le faisceau laser UV mis en forme (40) a une fluence comprise dans les plages suivantes : de 50 mJ/cm² à 400 mJ/cm² ou de 150 mJ/cm² à 250 mJ/cm² sur le tissu du patient.

16. Système laser UV ophtalmologique (1) selon l'une des revendications 1 à 15, comprenant en outre un scanner (5) disposé en aval du convertisseur de fréquence (3) configuré pour diriger le faisceau laser UV (30) sur une pluralité de positions sur le tissu d'un patient selon un modèle prédéterminé.

17. Système laser UV ophtalmologique (1) selon l'une des revendications 1 à 16, comprenant en outre un sélecteur de longueur d'onde (6) disposé en aval du convertisseur de fréquence (3) configuré pour laisser passer une plage de longueurs d'ondes particulière du faisceau laser UV (30) tout en redirigeant le reste du faisceau laser UV (30) sur un absorbeur de faisceau (61).

18. Système laser UV ophtalmologique (1) selon l'une des revendications 1 à 17, comprenant en outre un module de diagnostic d'impulsion (7) placé en aval du convertisseur de fréquence (3) et configuré pour mesurer la puissance du faisceau laser UV (30).
